# EUROPÄISCHE PATENTSCHRIFT

(11) **EP 3 280 484 B1**
(45) Veröffentlichungstag und Bekanntmachung des Hinweises auf die Patenterteilung: **16.10.2024**
(21) Anmeldenummer: 16720725.7
(22) Anmeldetag: 07.04.2016
(51) Int. Cl.: A61M 37/00

(54) **MIKRONADELSYSTEM ZUR APPLIKATION VON FLÜSSIGEN FORMULIERUNGEN**
MICRO-NEEDLE SYSTEM FOR APPLICATION OF LIQUID FORMULATIONS
SYSTÈME DE MICRO-AIGUILLES DESTINÉ À L'APPLICATION DE FORMULES LIQUIDES

(30) Priorität: 07.04.2015 EP 15162636
(43) Veröffentlichungstag der Anmeldung: 14.02.2018
(73) Patentinhaber: LTS Lohmann Therapie-Systeme AG, 56626 Andernach (DE)
(72) Erfinder: HEUSER, Karsten, 53498 Bad Breisig (DE); SPILGIES, Heiko, 56068 Koblenz (DE)
(74) Vertreter: dompatent von Kreisler Selting Werner - Partnerschaft von Patent- und Rechtsanwälten mbB
(86) Internationale Anmeldenummer: PCT/EP2016/057675
(87) Internationale Veröffentlichungsnummer: WO 2016/162449

(56) Entgegenhaltungen:
- WO-A1-2014/059104
- US-A1- 2003 187 395
- US-A1- 2011 276 027
- US-A1- 2012 109 065

## Beschreibung

Die vorliegende Erfindung betrifft ein Mikronadelsystem (kurz: MNS) zur intradermalen Applikation von Lösungen oder Formulierungen. Die Erfindung betrifft ein Mikronadelsystem umfassend ein Abdeckelement **(1),** einen Wirkstoffcontainer **(2),** einen Rahmen **(3),** einen Mikronadelarray (kurz: MNA) **(4)** und einer Grundplatte **(5),** wobei das Mikronadelarray (MNA) **(4)** mit dem Rahmen **(3)** verbunden ist, und die Grundplatte **(5)** eine Öffnung zur Aufnahme des Mikronadelarrays **(4)** aufweist, das Abdeckelement **(1)** und die Grundplatte **(5)** über ein Gewinde beweglich miteinander verbunden sind, die Grundplatte **(5)** mit einem Fixierpflaster **(6)** verbunden ist, und der Rahmen **(3)** und die Grundplatte **(5)** linear gegeneinander verschiebbar sind, und die Grundplatte **(5)** mindestens ein Öffnungselement **(8)** zur Öffnung des Wirkstoffcontainers **(2)** aufweist.

Das erfindungsgemäße MNS ist zum intradermalen Verabreichen von Arzneimitteln, Wirkstoffen, pharmazeutischen oder kosmetischen Zusammensetzungen oder anderen Substanzen an ein Individuum, vorzugsweise an einem Patienten über einen längeren Zeitraum geeignet.

### Hintergrund der Erfindung

Mikronadelsysteme und Vorrichtungen bei denen Mikronadelarrays zur schmerzfreien intradermalen Verabreichung von Arzneimitteln verwendet werden, sind im Stand der Technik bekannt.

Die Haut besteht aus mehreren Schichten. Die äußerste Hautschicht, das *Stratum Corneum,* weist bekannte Sperreigenschaften auf, um ein Eindringen von Fremdsubstanzen in den Körper und ein Austreten von Eigensubstanzen aus dem Körper zu verhindern. Das Stratum Corneum, das eine komplexe Struktur aus kompaktierten keratotischen Zellresten mit einer Dicke von ungefähr 10-30 Mikrometern ist, bildet hierzu eine wasserdichte Membran zum Schutz des Körpers aus. Die natürliche Undurchlässigkeit des Stratum Corneum verhindert das Verabreichen der meisten pharmazeutischen Mittel und anderer Substanzen durch die Haut im Rahmen einer intradermalen Applikation.

Das Verabreichen verschiedener Substanzen erfolgt daher beispielsweise durch Erzeugen von Mikroporen oder Schnitten im Stratum Corneum und Zuführen bzw. Applikation eines Arzneimittels in oder unter das Stratum Corneum. Auf diese Weise können zahlreiche Arzneimittel z.B. auch subkutan oder intradermal bzw. intrakutan verabreicht werden.

Mikronadelsysteme (MNS), die aus einem Mikronadelarray (MNA) und gegebenenfalls weiteren Komponenten bestehen, benötigen zur Anwendung ein Element, welches die Mikronadeln (auch: Hautdurchdringungselemente) des Arrays (MNA) gegen die Applikationsstelle auf der Haut presst, um das Stratum Corneum zu durchdringen und auf diese Weise einen Fluidkanal zwischen dem externen Arzneimittelreservoir (z.B. Container) und der Haut über den MNA herzustellen. Wird für das Arzneimittel eine flüssige Formulierung gewählt, so ist im MNS ein Element erforderlich, das den Wirkstoffcontainer öffnet und je nach Ausführung auch das Arzneimittel aus dem Wirkstoffcontainer drückt. Für die Realisierung des erstgenannten Elements sind verschiedene Ausführungsformen bekannt, die in der Regel einen mechanischen Energiespeicher zur Öffnung des Wirkstoffcontainers nutzen. Das Ausdrücken aus dem Wirkstoffcontainer übernimmt z.B. eine in das MNS integrierte Spritze oder miniaturisierte Pumpe.

Ein entsprechend einfaches Mikronadelsystem ist in WO 02/05889 A1 beschrieben. Diese Vorrichtung umfasst ein Gehäuse mit einem innenliegenden Wirkstoffcontainer in Form einer flexiblen Blase. Die flexible Blase ist in einem Hohlraum in dem Gehäuse positioniert. Der Hohlraum ist von einem Abdeckteil abgedeckt, das nach unten gedrückt werden kann, um die flexible Blase gegen ein auf dem Boden des Gehäuses befindliches Mikronadelarray zu pressen. Dadurch wird der Wirkstoffcontainer geöffnet, und die in der flexiblen Blase enthaltene Flüssigkeit fließt zu mehreren Mikronadeln.

DE 603 05 901 T2 offenbart eine Vorrichtung, die aus einem Gehäuse und einer Kartusche besteht. Die Kartusche beinhaltet den Container für die wirkstoffhaltige Lösung und weist neben der Bodenwand eine von der Bodenwand beabstandete untere Außenwand mit einem integrierten Nadelarray auf. Das Gehäuse umfasst ein Basisteil, eine umlaufende Seitenwand und ein Abdeckteil, das zwischen einer offenen und einer geschlossenen Position schwenkbar ist und mit dem Basisteil über ein Scharnier, einen Schnapp-, Press- oder Reibsitz verbunden ist. Das Mikronadelsystem wird durch Positionieren der Kartusche in dem Gehäuse zusammengesetzt, wobei die Kartusche in dem Gehäuse mittels einer Nut in einer definierten Position ausgerichtet ist. Das Mikronadelsystem wird auf der Hautoberfläche des Patienten derart positioniert, dass das Nadelarray die Oberfläche der Haut durchsticht, bevor das Abdeckteil des Gehäuses in die geschlossene Position geschwenkt wird. Beim Schließen des Abdeckteils wird der Wirkstoffcontainer durchstochen. Das Abdeckteil des Gehäuses weist eine Feder zum Aufbringen eines Drucks auf die Kartusche auf und bewirkt, dass die wirkstoffhaltige Lösung bei geschlossenem Abdeckteil abgegeben wird. Weiterhin ist zum Fixieren ein Armband erforderlich.

WO 2014/059104 A1 betrifft generisch ein MNS mit Abdeckelement, Grundplatte, Rahmen und Fixierpflaster, jedoch kann nicht mit Hilfe eines Gewindes über die Öffnung der Grundplatte von Lager- in die Applikationsposition mit Kraftschluss übergegangen werden.

Die im Stand der Technik bekannten Vorrichtungen und Verfahren zur intradermalen Applikation von Wirkstoffen sind lediglich in begrenztem Maße erfolgreich.

Die bekannten Mikronadelsysteme haben den Nachteil, dass sie nur während der kurzen Anwendung (einige Sekunden oder weniger) eine Kraft auf das MNA ausüben und das MNA in der darauffolgenden Anwendungsphase (einige Minuten bis einige Tage) dazu neigt, sich wieder aus der Haut zu lösen. Für viele Anwendungen ist es jedoch erforderlich, einen dauerhaften Kraftschluss zur Haut während der Applikation beziehungsweise über einen längeren Zeitraum zu gewährleisten.

Viele bekannte Mikronadelsysteme haben zudem den Nachteil, dass Applikatoren notwendig sind, die nicht Bestandteil des Mikronadelsystems sind, sondern als zusätzliche getrennte Einheit bereitgestellt werden müssen. Wünschenswert für die intradermale Applikation ist eine Ausführung, die MNS und Applikationssystem in einer Einheit kombiniert, wobei die Bauform des Gesamtsystems - insbesondere die Bauhöhe und der Durchmesser - ein komfortables Anwenden bzw. Tragen des MNS nicht beeinflusst.

Alle bekannten Vorrichtungen mit flüssigem Wirkstoffcontainer bestehen zudem aus einer Kombination eines Mikronadelarrays mit einer Spritze, einer Pumpe oder Feder zum Abgeben der wirkstoffhaltigen Lösung. Diese Vorrichtungen sind aufgrund ihrer Bauform unkomfortabel in der Anwendung und aufwändig in der Herstellung. Es besteht Bedarf an Vorrichtungen, die einfach herzustellen und anzuwenden sind.

Es besteht daher die Aufgabe ein MNS mit verbessertem Kraftschluss bereitzustellen.

Diese Aufgabe wird erfindungsgemäß durch ein Mikronadelsystem (MNS) nach Anspruch 1 und Unteransprüchen gelöst.

In dem erfindungsgemäßen MNS ist das Mikronadelarray **(4)** mit dem Rahmen **(3),** vorzugsweise an der Unterseite des Rahmens **(3)** verbunden und die Grundplatte **(5)** weist eine Öffnung zur Aufnahme des Mikronadelarrays **(4)** auf.

Der Rahmen **(3)** kann ebenfalls als eine Halterung für das Mikronadelarray **(4)** angesehen werden, und dient zur Fixierung des Mikronadelarrays **(4)** als auch zur Aufnahme eines Wirkstoffcontainers **(2).**

Das Abdeckelement **(1)** und die Grundplatte **(5)** sind beweglich über ein Gewinde miteinander verbunden. Die Grundplatte **(5)** ist ferner ganz oder teilweise mit einem Fixierpflaster **(6)** verbunden oder versehen und der Rahmen **(3)** und die Grundplatte **(5)** sind linear gegeneinander verschiebbar.

Das MNS ist vorzugsweise bis zur Anwendung durch eine Schutzfolie **(7),** insbesondere auf dem Fixierpflaster **(6)** geschützt.

Das erfindungsgemäße MNS besteht somit, im Gegensatz zu den im Stand der Technik bekannten Mikronadelsystemen, im Wesentlichen aus einem Wirkstoffcontainer, der in einer Einheit über ein Fixierpflaster mit der Haut verbunden werden kann. Das erfindungsgemäße MNS erfordert keinen zusätzlichen Applikator, keine Pumpe, Spritze oder Feder. Es ist einfach und kostengünstig herzustellen. Das MNS kann, muss aber nicht, durch weitere Bestandteile ergänzt werden. Es ist zum intradermalen Verabreichen von Arzneimitteln, Wirkstoffen, pharmazeutischen oder kosmetischen Zusammensetzungen oder anderen Substanzen an ein Individuum, vorzugsweise an einem Patienten über einen längeren Zeitraum geeignet. Die Handhabung des erfindungsgemäßen MNS ist einfach und kann beispielsweise im Rahmen der Selbstmedikation durch den Patienten als auch bei kosmetischer Anwendung durch das Individuum selbst zu Hause bzw. am Point-of-Care durchgeführt werden. Insbesondere das Einstechen der Mikronadeln in die Haut ist ein routinemäßiger Vorgang und Bedarf nicht der ärztlichen Kontrolle und Überwachung oder Aufsicht durch Fachpersonal. Dies ist insbesondere bei längeren oder wiederholt durchzuführenden Applikationen vorteilhaft, da der Patient bzw. das Individuum nicht wiederholt bzw. über den längeren Zeitraum durch Fachpersonal betreut werden muss. Dies ist möglich, da die Nadeln des MNA einfach in das Stratum Corneum eingebracht werden können und dort, auch ohne Nachdrücken, über einen längeren Zeitraum verbleiben, so dass die Applikation der gewünschten Menge an Arzneimitteln, Wirkstoff, pharmazeutischer Zusammensetzung oder anderer Substanzen, auch durch den Patienten bzw. das Individuum selbst, mittels des erfindungsgemäßen MNS sichergestellt ist.

Gegenstand der Erfindung ist daher ein Mikronadelsystem zur intradermalen Applikation umfassend
i.) ein Abdeckelement **(1)** über einen Wirkstoffcontainer **(2),** wobei der Wirkstoffcontainer **(2)** in einem Rahmen **(3)** aufweisend einen Mikronadelarray **(4)** aufgenommen ist, und
ii.) eine Grundplatte **(5),** wobei die Grundplatte **(5)** eine Öffnung zur Aufnahme des Mikronadelarrays **(4)** aufweist, und das Abdeckelement **(1)** und die Grundplatte **(5)** über ein Gewinde beweglich miteinander verbunden sind,
iii.) die Grundplatte **(5)** ganz oder teilweise mit einem Fixierpflaster **(6)** verbunden ist, und
iv.) der Rahmen **(4)** und die Grundplatte **(6)** beweglich miteinander verbunden sind, wobei der Rahmen **(4)** aufweisend einen Mikronadelarray **(5)** in Richtung der Öffnung der Grundplatte **(6)** linear bewegt werden kann, und die Grundplatte **(5)** mindestens ein Öffnungselement **(8)** zur Öffnung des Wirkstoffcontainers **(2)** aufweist.

Ferner ist das Abdeckelement **(1)** an der Grundplatte **(5)** über ein Gewinde beweglich miteinander verbunden.

Der Begriff "intradermale Applikation (synonym: "intrakutane Applikation") beschreibt erfindungsgemäß die Verabreichung von Stoffen über das MNA in die Haut und erfordert ein Einstechen der Mikronadeln in die Haut.

Erfindungsgemäß wird nach Fixierung auf der Haut mittels eines Fixierpflasters **(6)** des Mikronadelsystems in einem ersten Schritt das Abdeckelement **(1)** an der Grundplatte **(5)** über das Gewinde gedreht und auf diese Weise der Rahmen **(4)** aufweisend einen Mikronadelarray **(4)** in Richtung der Öffnung der Grundplatte **(5)** bewegt.

Im Zuge dieser Bewegung oder Hub erreicht der Rahmen **(3)** aufweisend einen Mikronadelarray **(4)** die Ebene der Öffnung der Grundplatte **(6),** wobei die überstehenden Mikronadeln in die Haut eindringen. Zugleich erreicht der mitgeführte Wirkstoffcontainer **(2)** ein oder mehrere Öffnungselemente **(8)** (Dorn, etc.), so dass der Container aufbricht und die Lösung oder Formulierung abgegeben wird, und zwar in das vorgesehene MNA. Die Öffnungselemente **(8)** sind in der Weise angebracht, dass die Öffnungselemente **(8)** den Wirkstoffcontainer **(2)** berühren und öffnen, sobald das Mikronadelarray **(4)** die Ebene der Öffnung der Grundplatte **(6)** erreicht (sogenannte Applikationsposition).

In einer bevorzugten Ausführungsform der Erfindung weist der Rahmen **(3)** des Mikronadelsystems mindestens einen oder mehrere Rasthaken **(9)** auf, die vorzugsweise überstehen und in die Grundplatte **(5)** greifen können. Die Grundplatte **(5)** kann entsprechende Aussparungen **(10)** aufweisen. Weiterhin dienen die Rasthaken **(9)** als Angriffspunkte für den Hub aus der oben genannten Drehbewegung des Abdeckelements **(1)** zur Grundplatte **(5)** .

Die Rasthaken **(9)** geben dem Rahmen **(3)** zur Grundplatte **(5)** eine definierte Position und verhindern auch eine (vollständige) Rotation des Rahmens **(3)** bzw. auf der Grundplatte **(5).** Gleichzeitig sind die Rasthaken am Rahmen **(3)** und die Aussparungen **(10)** in der Grundplatte **(5)** so ausgebildet, dass der Rahmen **(3)** in der Grundplatte **(5)** beweglich montiert ist.

Befindet sich der Rahmen **(3)** in der Ebenen der Grundplatte **(5)** bzw. Fixierpflaster **(6),** dann ist das MNA **(4)** in der Öffnung der Grundplatte **(5)** lokalisiert.

Der Rahmen **(3)** und die Grundplatte **(5)** sind beweglich miteinander verbunden, jedoch linear gegeneinander verschiebbar, wobei der oder die Rasthaken **(9)** eine Rotationsbewegung oder Drehung des Rahmens **(3)** auf der Grundplatte **(5)** verhindern sollen.

Wie bereits zum Mikronadelsystem beschrieben, sind das Abdeckelement **(1)** und die Grundplatte **(5)** über ein Gewinde beweglich miteinander verbunden. In einer bevorzugten Ausführungsform des Mikronadelsystems wird der Wirkstoffcontainer durch eine Viertel-Gewindeumdrehung geöffnet. Der bevorzugte Drehwinkel beträgt 90°. Der Drehwinkel kann je nach Ausführung zwischen 10° und 350° betragen. Das Erreichen des maximalen Drehwinkels kann dem Anwender angezeigt werden, im einfachsten Fall z.B. durch eine Positionsmarkierung im Anschlag.

In einer weiteren bevorzugten Ausführungsform des Mikronadelsystems ist das Fixierpflaster **(6)** durch eine Schutzfolie **(7)** geschützt.

In einer weiteren bevorzugten Ausführungsform des Mikronadelsystems beträgt der Durchmesser des Mikronadelsystems mindestens 10 mm, vorzugsweise mindestens 25 mm, besonders bevorzugt mindestens 50 mm.

In einer weiteren bevorzugten Ausführungsform des Mikronadelsystems beträgt die Höhe des Mikronadelsystems maximal 30 mm, vorzugsweise maximal 20 mm, besonders bevorzugt maximal 10 mm.

In einer weiteren bevorzugten Ausführungsform des Mikronadelsystems ist der Wirkstoffcontainer **(2)** ein Blow-Fill-Seal-Container oder Einwegcontainer.

In einer weiteren bevorzugten Ausführungsform des Mikronadelsystems umfasst der Wirkstoffcontainer **(2)** ein oder mehrere Stoffe, Arzneimittel, Wirkstoffe, Lösungen oder (Flüssig-)Formulierungen, insbesondere pharmazeutische Wirkstoffe oder pharmazeutische Zusammensetzungen, insbesondere Antibiotika, antivirale Wirkstoffe, Analgetika, Anästhetika, Anorexika, Antiarthritika, Antidepressiva, Antihistaminika, entzündungshemmende Mittel, antineoplastische Mittel, Impfstoffe, einschließlich DNA-Impfstoffe, und dergleichen, Proteine, Peptide oder Fragmente davon, Nukleinsäuren oder Teile davon, als auch Kosmetika, Nahrungsergänzungsmittel, Sonnenschutzmittel, Insektenschutzmittel, Radikalfänger, Hydratisierungsmittel, Farbstoffe.

Bevorzugt enthält der Wirkstoffcontainer **(2)** Lösungen oder (Flüssig-)Formulierungen umfassend Wirk- und Hilfsstoffe.

Der Begriff der "Lösung" oder "(Flüssig-)Formulierung" bedeutet, dass es sich um einen oder mehrere Stoffe handelt, welche zumindest einen solchen Aggregatzustand aufweist, dass er bei Raumtemperatur in einer vorgegebenen Applikationszeit durch das MNA **(4)** intradermal appliziert werden kann. Geeignete Viskositäten sind Werte zwischen 0 und 200 mPa*s.

Gegenstand der Erfindung ist ebenfalls ein Verfahren zur Herstellung eines erfindungsgemäßen Mikronadelsystems umfassend die Schritte
a) Bereitstellung eines Wirkstoffcontainers **(2),** vorzugsweise mittels Herstellung im Blow-Seal-Verfahren,
b) Bereitstellung eines Mikronadelarrays **(4),** und
c) der Wirkstoffcontainer **(2)** und das Mikronadelarray **(4)** mit dem Rahmen **(3),** der Grundplatte **(5)** und dem Fixierpflaster **(6)** zum Mikronadelsystem verbunden werden, wobei das Abdeckelement **(1)** und die Grundplatte **(5)** über ein Gewinde beweglich miteinander verbunden sind und durch Drehung des Abdeckelements **(1)** zur Grundplatte **(5)** der Wirkstoffcontainer **(2)** durch ein Öffnungselement **(8)** geöffnet wird.

Ebenfalls ist Gegenstand der Erfindung die Herrichtung von ein oder mehreren Stoffen, Arzneimitteln, Wirkstoffen, Lösungen oder (Flüssig-)Formulierungen zur intradermalen Applikation in einem erfindungsgemäßen Mikronadelsystem.

Das erfindungsgemäße MNS kann einen oder mehrere MNA **(4)** und einen oder mehrere Wirkstoffcontainer **(2)** umfassen. Das erfindungsgemäße MNS ermöglicht ein schmerzfreies Durchstechen des Stratum Corneums und ein Eindringen der Mikronadeln in die Haut mit geringem Kraftaufwand. Das erfindungsgemäße MNS ermöglicht besonders vorteilhaft einen konstanten Kraftschluss mit der Haut und eine Aufrechterhaltung eines konstanten und dauerhaften Kraftschlusses zwischen Haut und MNA **(4)** während der Applikation. Das erfindungsgemäße MNS ist in der Weise ausgebildet, dass es bei der Anwendung zunächst auf der Haut aufgebracht und fixiert wird. Das MNA **(4)** befindet sich in einer ersten Position - die Lagerposition. Durch Drehung des Abdeckelements **(1)** um die Grundplatte **(5)** wird der Rahmen **(3)** linear gegen die Grundplatte **(5)** verschoben (Applikationsmechanismus). Dabei wird das MNA **(4)** aus der Lagerposition in die Applikationsposition platziert und gleichzeitig wird der Wirkstoffcontainer **(2)** durch ein Öffnungselement **(8)** geöffnet. Die im Wirkstoffcontainer **(2)** enthaltene Lösung fließt aus dem Wirkstoffcontainer **(2)** in den MNA. bzw. in die Mikronadeln. In der Applikationsposition haben die Mikronadeln das Stratum Corneum durchstochen und sind in die Haut eingedrungen. Die Flüssigkeit wird durch die Mikronadeln intradermal appliziert. Durch Betätigung des Applikationsmechanismus wird eine konstante Spannung zwischen Haut und MNS erzeugt und aufrechterhalten. Dadurch besteht während der gesamten intradermalen Applikation ein dauerhafter Kraftschluss zwischen dem MNS und der Haut, so dass die Lösung auch über einen längeren Zeitraum verabreicht werden kann. Das MNA **(4)** verbleibt während der gesamten Applikation in der Haut, ohne dass sich die Nadeln des MNA **(4)** aus der Haut lösen.

Das Abdeckelement **(1)** weist eine Form und Abmessungen auf, die zu denen der Grundplatte **(5)** komplementär sind. Das Abdeckelement **(1)** und die Grundplatte **(5)** bilden ein Gehäuse aus. Vorzugsweise weist das Abdeckelement **(1)** ein Gewinde zum Verriegeln des Abdeckelements **(1)** in einer geschlossenen Position und zur Überführung des MNA von der Lagerposition in die Applikationsposition auf.

Das Abdeckelement **(1)** weist eine obere Seite und eine Bodenseite auf, wobei die Bodenseite nach innen gerichtet der Grundplatte **(5)** zugewandt ist und vorzugsweise mit den Rasthaken **(9)** in Kontakt ist. Das Abdeckelement **(1)** hat beispielsweise die Form einer gewölbten Kappe. Vorzugsweise hat das Abdeckelement **(1)** an der oberen Seite eine Struktur, die die Überführung von der Lagerposition in die Applikationsposition erleichtert. Beispielsweise weist das Abdeckelement **(1)** an der oberen Seite ein oder mehrere Rillen auf, die ein Abrutschen verhindern. Weiterhin kann das Abdeckelement **(1)** ein oder mehrere Markierungen aufweisen, die vorgeben, wie das MNS aus der Lagerposition in die Applikationsposition überführt werden kann. Die Bodenseite weist ein umlaufendes Gewinde auf, das zu dem Gewinde der Grundplatte **(5)** komplementär ist, so dass Abdeckelement **(1)** und Grundplatte **(5)** durch Gewindeumdrehungen verbunden werden können. Gleichzeitig bewirkt die Drehung des Gewindes zwischen Abdeckelement **(1)** und Grundplatte **(5)** die Überführung des MNA von der Lagerposition in die Applikationsposition.

Das Abdeckelement **(1)** und die Grundplatte **(5)** bilden ein Gehäuse aus mit einem innenliegenden Hohlraum, der zum Aufnehmen des Rahmens **(3),** des MNA **(4)** und des Wirkstoffcontainers **(2)** geeignet ist.

Die Grundplatte **(5)** weist einen zum Grundplattenrand nach innen versetzten umlaufenden Überstand samt Öffnung auf. Dieser Überstand dient als Abstandshalter zur Erzeugung des Hohlraums. Auf der äußeren Seite des Randes befindet sich ein Gewinde, das zum Gewinde des Abdeckelements **(1)** komplementär ist. Zur Erzeugung des Hohlraums wird das Abdeckelement **(1)** auf die Grundplatte **(5)** aufgesetzt und keine Gewindeumdrehung durchgeführt. Durch eine teilweise oder ganze Gewindeumdrehung oder durch mehrere Gewindeumdrehungen wird der durch das Abdeckelement **(1)** und die Grundplatte **(5)** begrenzte Hohlraum verkleinert.

In der Lagerposition befindet sich das MNA **(4)** oberhalb der Haut, die Mikronadeln sind nicht in das Stratum Corneum eingedrungen. Beim Übergang in die Applikationsposition wird der Hohlraum verkleinert und das MNA **(4)** abgesenkt. Dabei dringen die Mikronadeln in die Haut ein, vorzugsweise in das Stratum Corneum bzw. durch das Stratum Corneum hindurch und verbleiben in dieser Position für die Dauer der Applikation bzw. die Dauer der Anwendung des MNS.

Der Rahmen **(3)** ist so dimensioniert, dass er den Wirkstoffcontainer **(2)** aufnehmen kann. Vorzugsweise wird der Wirkstoffcontainer **(2)** in den Rahmen **(4)** derartig eingelegt oder fixiert, dass er bei der Gewindedrehung ebenfalls linear gegenüber der Grundplatte **(6)** verschoben wird.

Der Wirkstoffcontainer **(2)** besteht aus einem Material, das durch mindestens ein Öffnungselement **(8)** geöffnet werden kann. In bevorzugten Ausführungsformen der Erfindung wird der Wirkstoffcontainer **(2)** im Blow-Fill-Seal-Verfahren hergestellt. Entsprechende Verfahren sind im Stand der Technik bekannt und beispielsweise in Andrew. W. Goll (ISPE (2012) Knowledge Brief, KB-0025-Jun12) beschrieben. Besonders bevorzugte Ausführungsformen des erfindungsgemäßen MNS betreffen solche, in denen ein Blow-Fill-Seal-Container verwendet wird.

Das erfindungsgemäße MNS umfasst ferner ein Fixierpflaster **(6)** zur Befestigung des MNS an der Haut. Der Begriff Fixierpflaster **(6)** umfasst dabei jedes Befestigungsmittel, das geeignet ist, das MNS an der Hautoberfläche zu befestigen. Das Fixierpflaster **(6)** kann beispielsweise einen festen Träger und eine chemische oder biologische Substanz umfassen,
vorzugsweise einem Klebstoff. Das Fixierpflaster **(6)** kann auch lediglich eine oder mehrere chemische und/oder biologische Substanzen umfassen bzw. daraus bestehen, wobei die eine oder mehrere chemischen und/oder biologischen Substanzen direkt auf die Unterseite der Grundplatte **(5)** aufgebracht werden und zur Befestigung des MNS an der Hautoberfläche geeignet sind. Das Fixierpflaster **(6)** kann ebenfalls eine strukturierte Oberfläche aufweisen oder daraus bestehen, beispielsweise eine Nanostruktur, die zur Befestigung des NMS an der Hautoberfläche geeignet ist. Entscheidend ist, dass durch das Fixierpflaster **(6)** eine entsprechend starke Befestigung und Fixierung auf der Hautoberfläche erzeugt wird, so dass ein konstanter Kraftschluss zwischen Haut und MNA **(4)** erzeugt wird und dieser Kraftschluss zwischen Haut und MNA **(4)** während der Applikation bestehen bleibt. Die Wahl des geeigneten Fixierpflasters **(6)** hängt von der Spannung der Haut in dem betreffenden Bereich (beispielsweise Bauch oder Rücken), dem Anteil an Fettgewebe in und unter dem Stratum Corneum in dem betreffenden Bereich, dem Body-Mass-Index des Individuums, dem Alter des Individuums, den Ernährungsgewohnheiten des Individuums (z.B. der Aufnahme von Flüssigkeit), den Lebensgewohnheiten des Individuums (z.B. der Sonnenbelastung der betreffenden Hautregion) und gegebenenfalls weiteren Faktoren ab. Entsprechend können vom Fachmann jeweils geeignete Fixierpflaster **(6)** für verschiedene Individuen oder Patienten und/oder Hautregionen ausgewählt werden. Des Weiteren hängt die Wahl des Fixierpflasters **(6)** von der Größe des MNS und der im MNS verwendeten Materialien bzw. deren Gewicht ab. Beispiele für geeignete Fixierpflaster **(6)** sind übliche kommerziell erhältliche (Haft)Kleber auf einen Träger, ggfs. auch doppelseitig beschichtet.

Das Fixierpflaster **(6)** ist fest mit der Grundplatte **(5)** verbunden. In einer besonderen Ausführungsform ist das Fixierpflaster **(6)** Bestandteil der Grundplatte **(5).** Das Fixierpflaster **(6)** weist eine oder mehrere Aussparungen auf, durch die die Mikronadeln des MNA **(4)** beim Einstellen der Applikationsposition hindurchgeführt werden und während der Applikation hindurch ragen. Die ausgesparte Region des Fixierpflasters **(6)** kann bis zur Applikation bzw. der Fixierung auf der Haut gegebenenfalls durch eine Schutzfolie **(7)** oder ähnliches geschützt sein.

Das Mikronadelarray (MNA) **(4)** (synonym: Mikronadelpatch oder Mikronadelpad) trägt erfindungsgemäß ein oder mehrere Hautdurchdringungselemente. Bei einigen Ausführungsformen sind die Hautdurchdringungselemente in einem Array von Reihen und Spalten angeordnet, die um eine im Wesentlichen gleichmäßige Distanz voneinander beabstandet sind. Die tatsächliche Länge und Beabstandung der Hautdurchdringungselemente können von der zu verabreichenden Lösung und der Verabreichungsstelle am Körper des Individuums bzw. Patienten abhängen. Typischerweise sind die Hautdurchdringungselemente Nadeln, vorzugsweise Hohlnadeln, die auf oder an einem Träger fixiert sind und von diesem Träger vorstehen. Die Hautdurchdringungselemente sind in einem Array angeordnet, das zum Verabreichen einer wirksamen Menge einer Lösung über einen definierten Zeitraum durch die Haut eines Individuums / Patienten vorgesehen ist. Typischerweise weist das Mikronadelarray **(4)** eine Fläche von ungefähr 1 cm² bis ungefähr 10 cm² und vorzugsweise ungefähr 2-5 cm² auf.

Vorzugsweise handelt es sich bei den Hautdurchdringungselementen um Hohlnadeln, die jeweils einen axialen Durchgang und eine abgeschrägte spitz zulaufende äußere Spitze zum Durchstechen der Haut des Individuums / Patienten aufweisen. Die Hautdurchdringungselemente sind derart in Löchern in einem festen Träger angebracht, dass die Lösung aus dem Wirkstoffcontainer **(2)** durch die axialen Durchgänge der Hohlnadeln hindurchfließen kann. Die Hautdurchdringungselemente können mittels eines geeigneten Klebers oder eines Presssitzes in den Öffnungen des festen Trägers befestigt sein. Bei einer alternativen Ausführungsform können Mikro- Hautdurchdringungselemente einstückig mit dem festen Träger ausgebildet sein. Vorzugsweise weisen die Hautdurchdringungselemente jeweils eine abgeschrägte, spitz zulaufende Spitze und einen axialen Durchgang auf, um eine Fluidverbindung zwischen dem Wirkstoffcontainer (2) und einer intradermalen Stelle in der Haut des Patienten herzustellen. Die Hautdurchdringungselemente haben vorzugsweise eine Länge, die geeignet ist, die gewünschte Hautdurchdringungstiefe zu erreichen. Die Länge und Dicke der Hautdurchdringungselemente werden auf der Basis der zu verabreichenden Lösung und der Dicke der Haut und des Zielbereichs, in dem MNS angebracht wird, ausgewählt. Bei Ausführungsformen der Erfindung können die Hautdurchdringungselemente Mikronadeln, Mikroröhrchen, massive oder hohle Nadeln, Lanzetten und dergleichen sein. Bei einer bevorzugten Ausführungsform sind die Hautdurchdringungselemente Hohlnadeln oder -kanülen aus Edelstahl. Die Nadeln sind ungefähr 24 Gauge bis 50 Gauge und vorzugsweise ungefähr 30 Gauge bis ungefähr 36 Gauge groß und bei der am stärksten bevorzugten Variante ungefähr 34 Gauge groß. Kleinere Nadeln durchdringen die Hautoberfläche leichter als größere Nadeln und werden im Allgemeinen bevorzugt. Die Nadeln befinden sich im MNS an der Unterseite des Rahmens (3), um eine effektive Länge von ungefähr 50 Mikrometern bis ungefähr 5000 Mikrometern zu bieten. Bei einer anderen Ausführungsform weisen die Nadeln eine effektive Länge von ungefähr 500 Mikrometern bis ungefähr 3000 Mikrometern auf. Bei weiteren Ausführungsformen können die Nadeln eine effektive Länge im Bereich von ungefähr 1000 Mikrometern und 2000 Mikrometern aufweisen. Typischerweise haben die Nadeln eine effektive Länge von ungefähr 500 Mikrometern bis ungefähr 1000 Mikrometern.

Die Bestandteile des MNS, insbesondere das Abdeckelement (1), der Rahmen (3), die Grundplatte (5), ggf. das Trägermaterial des MNA (4) und / oder ggf. das Trägermaterial des Fixierpflasters (6) können aus einem geeigneten Kunststoffmaterial gefertigt sein. Typischerweise wird dafür ein nichtreagierendes, inertes, gut verträgliches, biokompatibles, beispielsweise dermatologisch getestetes Kunststoffmaterial verwendet. Geeignete Kunststoffmaterialien umfassen Polyethylen, Polypropylen, Polyester, Polyamide, Polycarbonate und Copolymere daraus.

In dem MNA **(4)** bilden die Hautdurchdringungselemente ein Array (d.h. eine Anordnung) beispielsweise in Form von voneinander beabstandeten Reihen und Spalten. Dabei können in dem MNA **(4)** ein, mindestens 2, mehrere oder viele Hautdurchdringungselemente vorgesehen sein, beispielsweise 10-1.000.000, vorzugsweise 50-100.000, besonders bevorzugt 100-10.000 Hautdurchdringungselemente. Das MNA **(4)** kann beispielsweise aus einem Silizium-Wafer hergestellt sein, das zur Ausbildung der einzelnen Nadeln maschinell bearbeitet und geätzt wird. Bei alternativen Ausführungsformen kann das NMA **(4)** aus Edelstahl, Wolframstahl und Legierungen aus Nickel, Molybdän, Chrom, Kobalt und Titan gefertigt sein. Bei weiteren Ausführungsformen kann das MNA **(4)** aus Keramikmaterialien, Glas, Polymeren und anderen nicht-reagierenden Materialien hergestellt sein.

Das Array von Hautdurchdringungselementen ist typischerweise in Reihen und Spalten angeordnet, die Hautdurchdringungselemente können jedoch in anderen geeigneten Mustern angeordnet sein. Vorzugsweise sind die Hautdurchdringungselemente in ausreichendem Maße voneinander beabstandet, damit die Hautdurchdringungselemente die Haut bis zu einer im Wesentlichen über das gesamte Array gleichmäßigen Tiefe durchdringen können ohne miteinander zu interferieren. Die bevorzugte Eindringtiefe der Nadeln ist dabei gegeben durch das Stratum Corneum, das vorzugsweise vollständig bzw. nahezu vollständig durchstochen wird. Bei bevorzugten Ausführungsformen dringen die Hautdurchdringungselemente in die Haut bis zu einer gleichmäßigen Tiefe ein und/oder durchstechen die Haut, um die Lösung in der gewählten Hauttiefe zu verabreichen und das Leckagerisiko beim Verabreichen der Substanz zu reduzieren. Die Anzahl von Hautdurchdringungselementen in dem Array kann je nach den Abmessungen der Hautdurchdringungselemente, der zu verabreichenden Substanz und der Durchdringtiefe variieren.

Das erfindungsgemäße MNS kann ein oder mehrere Wirkstoffcontainer **(2)** umfassen. Die Wirkstoffcontainer **(2)** können unterschiedliche Volumina haben bzw. dafür ausgelegt sein, mit unterschiedlichen Volumina an Lösung befüllt zu sein. Die Wahl des Volumens ist abhängig von dem zu verabreichenden (Wirk-)Stoff bzw. der zu verabreichenden Substanz, der gewählten Formulierung, der Dosierung, dem Therapieschema und anderen Faktoren. Die Applikationsdauer hängt vom Volumen des Wirkstoffcontainers **(2),** dem zu verabreichenden (Wirk-)Stoff, der gewählten Formulierung, der Dosierung, dem Therapieschema, dem gewählten MNA **(4)** (z.B. dem inneren Durchmesser der Hautdurchdringungselemente), der Beschaffenheit der Haut in dem betreffenden Bereich und anderen Faktoren ab. Beispielsweise ist das MNS für Applikationen von 0,5 min - 10 Tagen geeignet, vorzugsweise 5 min - 1 Tag, bevorzugt 1 - 5 Stunden, besonders bevorzugt etwa 2 Stunden. Während dieser Zeit ist durch das erfindungsgemäße MNS ein konstanter Kraftschluss zwischen der Haut und dem MNA **(4)** gewährleistet, ohne dass die Nadeln nachgedrückt werden müssen.

Beispielhaft wird eine Ausführungsform des erfindungsgemäßen MNS durch die nachfolgenden Figuren und Beispiele veranschaulicht.
Figur 1: Zeigt eine Explosionsansicht der Bestandteile des erfindungsgemäßen MNS. Das Gesamtsystem hat in der dargestellten exemplarischen Ausführung einen maximalen Durchmesser von 50 mm in den Bauteilen Fixierpflaster **(6)** und Schutzfolie **(7)** und eine Gesamthöhe von ca. 7 mm.
Figur 2: Gezeigt ist die Schnittansicht des MNS in der Lagerposition.

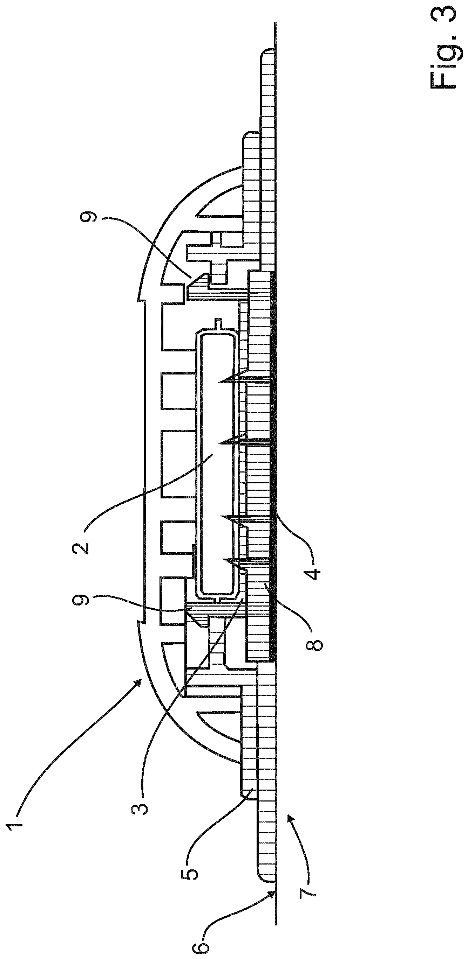

Figur 3: Gezeigt ist die Schnittansicht des MNS in der Applikationsposition.

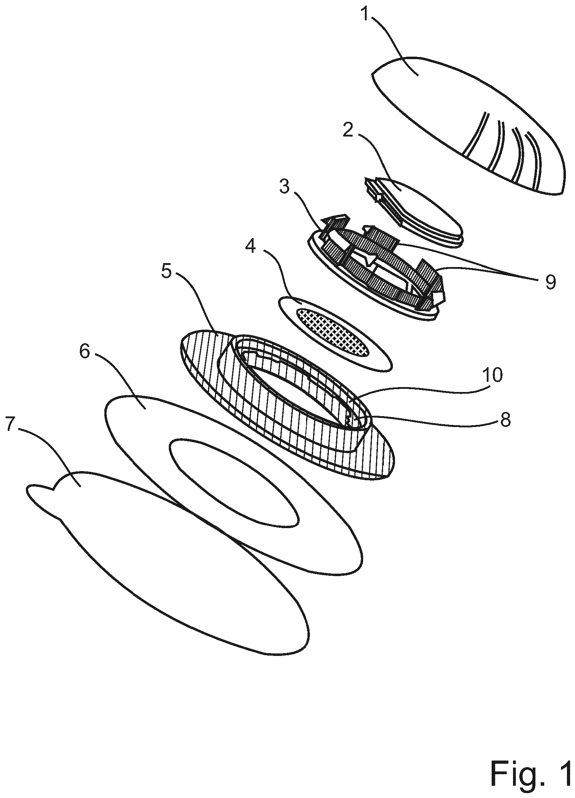

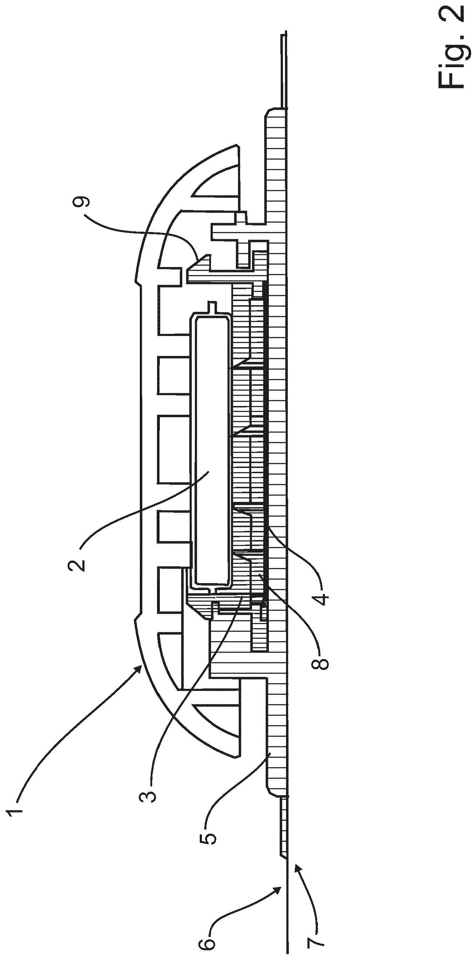

Beispiel 1: Aufbau eines MNS gemäß Figur 1:
Die Wirkstofflösung befindet sich in einem Blow-Fill-Seal-Container **(2),** der in einem Rahmen **(3)** liegt. Das MNA **(4)** ist mit der Unterseite des Rahmens **(3)** verbunden. Der Rahmen **(3)** ist in einer Grundplatte **(5)** beweglich montiert, dabei sind Rahmen **(3)** und Grundplatte **(5)** gegeneinander linear verschiebbar, die Rasthaken an dem Rahmen **(4)** definieren dabei den Hub und verhindern eine Rotationsbewegung. Die Grundplatte **(5)** ist fest mit dem Fixierpflaster **(6)** verbunden und durch eine Schutzfolie **(7)** bis zur Anwendung geschützt. Kappe **(3)** und Grundplatte **(5)** sind über ein Gewinde beweglich verbunden.
Beispiel 2: Anwendung des Applikationsmechanismus:
   Ausgehend von der Schnittansicht des MNS in der Lagerposition gemäß Figur 2 wird zunächst die Schutzfolie **(7)** entfernt und das MNS durch das Fixierpflaster **(6)** an der Haut fixiert. Durch eine Viertel-Gewindeumdrehung der Kappe **(3)** um die Grundplatte **(5)** werden der Blow-Fill-Seal-Container, der Rahmen **(3)** und das MNA **(4)** linear gegen die Grundplatte **(5)** verschoben und der Wirkstoffcontainer **(2)** durch eine Dorne (Öffnungselement **(8))** an der Grundplatte **(5)** geöffnet.

## Patentansprüche

1. Mikronadelsystem zur intradermalen Applikation umfassend
i.) ein Abdeckelement **(1)** über einen Wirkstoffcontainer **(2),** wobei der Wirkstoffcontainer **(2)** in einem Rahmen **(3)** aufweisend einen Mikronadelarray **(4)** aufgenommen ist, und
ii.) eine Grundplatte **(5),** wobei die Grundplatte **(5)** eine Öffnung zur Aufnahme des Mikronadelarrays **(4)** aufweist, und das Abdeckelement **(1)** und die Grundplatte **(5)** über ein Gewinde beweglich miteinander verbunden sind,
iii.) die Grundplatte **(5)** ganz oder teilweise mit einem Fixierpflaster **(6)** verbunden ist, und
iv.) der Rahmen **(3)** und die Grundplatte **(5)** beweglich miteinander verbunden sind, wobei der Rahmen **(3)** aufweisend einen Mikronadelarray **(4)** in Richtung der Öffnung der Grundplatte **(5)** linear bewegt werden kann, und die Grundplatte **(5)** mindestens ein Öffnungselement **(8)** zur Öffnung des Wirkstoffcontainers **(2)** aufweist.

2. Mikronadelsystem nach Anspruch 1 **dadurch gekennzeichnet, dass** der Rahmen **(3)** einen oder mehrere Rasthaken **(9)** aufweist, die in Aussparungen **(10)** in der Grundplatte **(5)** greifen können.

3. Mikronadelsystem nach einem der vorgehenden Ansprüche, **dadurch gekennzeichnet, dass** die Grundplatte **(5)** einen zum Grundplattenrand nach innen versetzten umlaufenden Überstand samt Öffnung aufweist und auf der äußeren Seite ein Gewinde aufweist, welches zum Gewinde des Abdeckelements (1) komplementär ist.

4. Mikronadelsystem nach einem der vorgehenden Ansprüche, **dadurch gekennzeichnet, dass** das Abdeckelement **(1)** auf der Bodenseite ein umlaufendes Gewinde aufweist, welches zu dem Gewinde der Grundplatte **(5)** komplementär ist.

5. Mikronadelsystem nach einem der vorgehenden Ansprüche, **dadurch gekennzeichnet, dass** das Abdeckelement **(1)** ein oder mehrere Markierungen aufweist, die vorgeben, wie das Mikronadelsystem aus der Lagerposition in die Applikationsposition überführt werden kann.

6. Mikronadelsystem nach einem der vorgehenden Ansprüche **dadurch gekennzeichnet, dass** das Fixierpflaster **(6)** durch eine Schutzfolie **(7)** geschützt ist.

7. Mikronadelsystem nach einem der vorgehenden Ansprüche **dadurch gekennzeichnet, dass** der Durchmesser des Mikronadelsystems mindestens 10 mm, vorzugsweise mindestens 25 mm, besonders bevorzugt mindestens 50 mm beträgt.

8. Mikronadelsystem nach einem der vorgehenden Ansprüche, **dadurch gekennzeichnet, dass** die Höhe des Mikronadelsystems maximal 30 mm, vorzugsweise maximal 20 mm, besonders bevorzugt maximal 10 mm beträgt.

9. Mikronadelsystem nach einem der vorgehenden Ansprüche, **dadurch gekennzeichnet, dass** der Wirkstoffcontainer **(2)** ein Blow-Fill-Seal-Container oder Einwegcontainer ist.

10. Mikronadelsystem nach einem der vorgehenden Ansprüche, **dadurch gekennzeichnet, dass** der Wirkstoffcontainer **(2)** ein oder mehrere Stoffe, Arzneimittel, Wirkstoffe, Lösungen oder (Flüssig-)Formulierungen umfasst.

11. Verfahren zur Herstellung eines Mikronadelsystems nach einem der Ansprüche 1 bis 10, wobei
a) ein Wirkstoffcontainer **(2)** bereitgestellt wird,
b) ein Mikronadelarray **(4)** bereitgestellt wird, und
c) der Wirkstoffcontainer **(2)** und das Mikronadelarray **(4)** mit dem Rahmen **(3),** der Grundplatte **(5)** und dem Fixierpflaster **(6)** zum Mikronadelsystem verbunden werden, **dadurch gekennzeichnet, dass** das Abdeckelement **(1)** und die Grundplatte **(5)** über ein Gewinde beweglich miteinander verbunden sind und durch Drehung des Abdeckelements **(1)** zur Grundplatte **(5)** der Wirkstoffcontainer **(2)** durch ein Öffnungselement **(8)** geöffnet wird.

12. Mikronadelsystems nach einem der Ansprüche 1 bis 10 aufweisend einen Wirkstoffcontainer **(2)** enthaltend Wirkstoffe in einer Flüssigformulierung zur Verwendung in der intradermalen Applikation.

## Claims

1. A microneedle system for intradermal delivery, comprising:
i.) a cover element (1) over an active substance container (2), wherein the active substance container (2) is accommodated in a frame (3) comprising a microneedle array (4); and
ii.) a base plate (5), wherein the base plate (5) has an opening for receiving the microneedle array (4), and the cover element (1) and the base plate (5) are movably connected to one another via a thread;
iii.) the base plate (5) is entirely or partially joined to a surgical tape (6); and
iv.) the frame (3) and the base plate (5) are movably connected to one another, wherein the frame (3) comprising a microneedle array (4) can be linearly displaced in the direction of the opening of the base plate (5), and the base plate (5) comprises at least one opening element (8) for opening the active substance container (2).

2. The microneedle system according to claim 1, wherein the frame (3) comprises one or more catch hooks (9), which can engage in recesses (10) in the base plate (5).

3. The microneedle system according to one of the preceding claims, wherein the base plate (5) has a peripheral projection, including an opening, which is inwardly offset with respect to the base plate edge, and has a thread on the outer side which is complementary to the thread of the cover element (1).

4. The microneedle system according to one of the preceding claims, wherein the bottom side of the cover element (1) comprises a peripheral thread, which is complementary to the thread of the base plate (5).

5. The microneedle system according to one of the preceding claims, wherein the cover element (1) comprises one or more markings, which define how the microneedle system can be transferred from the storage position into the delivery position.

6. The microneedle system according to one of the preceding claims. wherein the surgical tape (6) is protected by a protective film (7).

7. The microneedle system according to one of the preceding claims, wherein the diameter of the microneedle system is at least 10 mm, preferably at least 25 mm, particularly preferred at least 50 mm.

8. The microneedle system according to one of the preceding claims, wherein the height of the microneedle system is no more than 30 mm, preferably no more than 20 mm, particularly preferred no more than 10 mm.

9. The microneedle system according to one of the preceding claims, wherein the active substance container (2) is a blow-fill-seal container or a disposable container.

10. The microneedle system according to one of the preceding claims, wherein the active substance container (2) contains one or more substances, medicinal drugs, active agents, solutions or (liquid) formulations.

11. A method for producing the microneedle system according to one of claims 1 to 10, in which
a) an active substance container (2) is provided;
b) a microneedle array (4) is provided; and
c) the active substance container (2) and the microneedle array (4) are combined with the frame (3), the base plate (5) and the surgical tape (6) to form the microneedle system,
wherein the cover element (1) and the base plate (5) are movably connected to one another via a thread and a rotation of the cover element (1) relative to the base plate (5) causes the active substance container (2) to be opened by an opening element (8).

12. The microneedle system according to one of claims 1 to 10, comprising the active substance container (2), containing active substances in a liquid formulation for use in the intradermal delivery.

## Revendications

1. Système à micro-aiguilles pour application intradermique comprenant
i.) un élément de recouvrement (1) sur un contenant de substances actives (2), le contenant de substances actives (2) étant logé dans un cadre (3) comportant un réseau de micro-aiguilles (4), et
ii.) une plaque de base (5), la plaque de base (5) comprenant une ouverture pour recevoir le réseau de micro-aiguilles (4), et l'élément de recouvrement (1) et la plaque de base (5) étant reliés l'un à l'autre de manière mobile par l'intermédiaire d'un filetage,
iii.) la plaque de base (5) étant reliée totalement ou partiellement à un sparadrap de fixation (6), et
iv.) le cadre (3) et la plaque de base (5) étant reliés l'un à l'autre de manière mobile, le cadre (3) comportant un réseau de micro-aiguilles (4) pouvant être déplacé linéairement dans la direction de l'ouverture de la plaque de base (5), et la plaque de base (5) comprenant au moins un élément d'ouverture (8) pour l'ouverture du contenant de substances actives (2).

2. Système à micro-aiguilles selon la revendication 1, **caractérisé en ce que** le cadre (3) comprend un ou plusieurs crochets d'encliquetage (9) qui peuvent venir en prise dans des encoches (10) dans la plaque de base (5).

3. Système à micro-aiguilles selon l'une quelconque des revendications précédentes, **caractérisé en ce que** la plaque de base (5) comprend une saillie périphérique, avec une ouverture, décalée vers l'intérieur par rapport au bord de la plaque de base, et comprend sur le côté extérieur un filetage qui est complémentaire au filetage de l'élément de recouvrement (1).

4. Système à micro-aiguilles selon l'une quelconque des revendications précédentes, **caractérisé en ce que** l'élément de recouvrement (1) comprend sur le côté inférieur un filetage périphérique qui est complémentaire du filetage de la plaque de base (5).

5. Système à micro-aiguilles selon l'une quelconque des revendications précédentes, **caractérisé en ce que** l'élément de recouvrement (1) comprend un ou plusieurs repères qui spécifient la manière dont le système à micro-aiguilles peut être transféré de la position de stockage à la position d'application.

6. Système à micro-aiguilles selon l'une des revendications précédentes, **caractérisé en ce que** le sparadrap de fixation (6) est protégé par un film protecteur (7).

7. Système à micro-aiguilles selon l'une quelconque des revendications précédentes, **caractérisé en ce que** le diamètre du système à micro-aiguilles est d'au moins 10 mm, de préférence d'au moins 25 mm, de manière particulièrement préférée d'au moins 50 mm.

8. Système à micro-aiguilles selon l'une quelconque des revendications précédentes, **caractérisé en ce que** la hauteur du système à micro-aiguilles est au maximum de 30 mm, de préférence au maximum de 20 mm, de manière particulièrement préférée au maximum de 10 mm.

9. Système à micro-aiguilles selon l'une quelconque des revendications précédentes, **caractérisé en ce que** le contenant de substances actives (2) est un contenant de soufflage-remplissage-scellage ou un contenant jetable.

10. Système à micro-aiguilles selon l'une quelconque des revendications précédentes, **caractérisé en ce que** le contenant de substances actives (2) comprend une ou plusieurs substances, médicaments, substances actives, solutions ou formulations (liquides).

11. Procédé de production d'un système à micro-aiguilles selon l'une quelconque des revendications 1 à 10,
a) un contenant de substances actives (2) étant fourni,
b) un réseau de micro-aiguilles (4) étant fourni, et
c) le contenant de substances actives (2) et le réseau de micro-aiguilles (4) étant reliés au cadre (3), à la plaque de base (5) et au sparadrap de fixation (6) pour former le système à micro-aiguilles, **caractérisé en ce que** l'élément de recouvrement (1) et la plaque de base (5) sont reliés l'un à l'autre de manière mobile par un filetage et **en ce que** le contenant de substances actives (2) est ouvert par un élément d'ouverture (8) moyennant une rotation de l'élément de recouvrement (1) vers la plaque de base (5).

12. Système à micro-aiguilles selon l'une quelconque des revendications 1 à 10, comprenant un contenant de substances actives (2) contenant des substances actives dans une formulation liquide pour une utilisation en application intradermique.
